# EUROPEAN PATENT APPLICATION

(11) **EP 4 692 313 A1**
(43) Date of publication of application: **11.02.2026**
(21) Application number: 23963186.4
(22) Date of filing: 28.12.2023
(51) Int. Cl.: C12N 1/21, C12N 9/14, C12N 15/55, C12P 13/14

(54) **L-GLUTAMIC ACID-PRODUCING BACTERIUM AND METHOD FOR PRODUCING L-GLUTAMIC ACID**

(71) Applicant: AJINOMOTO CO., INC., Chuo-ku Tokyo 104-8315 (JP)
(72) Inventor: HAMANO KOSEKI, Chie, Kawasaki-shi, Kanagawa 210-8681 (JP); INOUE, Kota, Kawasaki-shi, Kanagawa 210-8681 (JP); ONISHI, Fumito, Kawasaki-shi, Kanagawa 210-8681 (JP)
(74) Representative: Hoffmann Eitle
(86) International application number: PCT/JP2023/047152
(87) International publication number: WO 2025/141838

(57) **Abstract**

To provide a method for producing L-glutamic acid and a bacterium for use in the method. L-glutamic acid is produced by culturing a coryneform bacterium having the ability to produce L-glutamic acid and modified to harbor a mutant acetyl CoA hydrolase gene encoding a mutant acetyl CoA hydrolase having the substitution of a serine residue at position 383 in the amino acid sequence of wild-type acetyl CoA hydrolase substituted with another amino acid residue in a culture medium, and collecting L-glutamic acid from the culture medium and/or cells of the bacterium.

## Description

### Technical Field

The present invention relates to the fermentation industry, and in particular, to a method for production of L-glutamic acid and bacterium used for the method.

### Background Art

L-amino acids are industrially produced, for example, by a fermentation method using microorganisms such as bacteria capable of producing L-amino acids (Non-Patent Document 1). For example, strains isolated from nature and their mutant strains are used as such microorganisms. In addition, the L-amino acid production ability of microorganisms can be improved by recombinant DNA technology.

Acetyl-CoA hydrolase is an enzyme that catalyzes the hydrolysis of acetyl-CoA to produce coenzyme A and acetic acid and/or the reverse reaction (Non-Patent Document 2). Production of L-glutamic acid, L-valine, and L-alanine are known to be enhanced by reducing the activity of this enzyme (Patent Document 1). However, it has not been so far that a specific mutation in the amino acid sequence of the enzyme contributes to the enhancement of L-glutamic acid production.

### Prior art references

### Patent documents

Patent Document 1: Patent Publication No. 2006-149214

### Non-patent documents

Non-Patent Document 1: Kunihiko Akashi et al. Amino Acid Fermentation, Gakkai Shuppan Center, pp. 195-215, 1986.
Non-Patent Document 2: KEGG (Kyoto Encyclopedia of Genes and Genomes) website: https://www.genome.jp/entry/3.1.2.1

### Summary of Invention

### Problem to be solved

An object of the present invention is to develop a novel technique to improve the L-glutamic acid production ability of a bacterium, and to provide an efficient method for producing L-glutamic acid and a bacterium for use in the method.

### Means to solve the problem

As a result of intensive research to solve the above problem, the inventors found that by modifying a coryneform bacterium so that the acetyl-CoA hydrolase has a specific mutation, it is possible to improve the L-glutamic acid production ability of the bacterium, thereby completed the invention.

The invention can be exemplified as follows
[1] A coryneform bacterium having L-glutamic acid producing ability, wherein said coryneform bacterium has been modified to harbor a mutant acetyl CoA hydrolase gene encoding a mutant acetyl CoA hydrolase having the substitution of a serine residue at position 383 in the amino acid sequence of the wild-type acetyl CoA hydrolase with another amino acid residue.
[2] The coryneform bacterium according to [1], wherein the other amino acid is lysine, glutamic acid, threonine, aspartic acid, asparagine, glutamine, arginine, cysteine, histidine, or methionine.
[3] The coryneform bacterium according to [1], wherein the other amino acid is cysteine.
[4] The coryneform bacterium according to any one of [1] to [3], wherein the wild-type acetyl CoA hydrolase is a protein as described in (a), (b) or (c) below.
   (a) a protein comprising the amino acid sequence shown in SEQ ID NO: 2;
   (b) a protein comprising an amino acid sequence including a substitution(s), a deletion(s), an insertion(s) and/or an addition(s) of 1 to 10 amino acid residues in the amino acid sequence shown in SEQ ID NO: 2, and having acetyl CoA hydrolase activity;
   (c) a protein comprising an amino acid sequence having 90% or more identity to the amino acid sequence shown in SEQ ID NO: 2 and having acetyl CoA hydrolase activity.
[5] The coryneform bacterium of any one of [1] to [4], wherein the coryneform bacterium is a bacterium belonging to the genus *Corynebacterium.*
[6] The coryneform bacterium of any one of [1] to [4], wherein said coryneform bacterium is *Corynebacterium glutamicum.*
[7] The coryneform bacterium of any one of [1] to [6], wherein said coryneform bacterium has been further modified to harbor a mutant yggB gene, wherein the mutant yggB gene is a gene encoding a protein comprising an amino acid sequence including a substitution(s), a deletion(s), an insertion(s) and/or an addition(s) of one or several amino acids in the amino acid sequence of SEQ ID NO: 8.
[8] The coryneform bacterium according to [7], wherein the mutant yggB gene is a gene encoding a protein comprising the amino acid sequence of SEQ ID NO: 10.
[9] A method for the production of L-glutamic acid, comprising
   culturing the coryneform bacteria of any one of [1] to [8] in a culture medium to accumulate L-glutamic acid in said medium and/or cells of the bacterium, and
   collecting L-glutamic acid from the culture medium and/or the cells of the bacterium.
[10] A mutant acetyl CoA hydrolase having the substitution of a serine residue at position 383 in the amino acid sequence of wild-type acetyl CoA hydrolase with another amino acid residue.
[11] The mutant acetyl CoA hydrolase of [10], wherein the other amino acid is lysine, glutamic acid, threonine, aspartic acid, asparagine, glutamine, arginine, cysteine, histidine or methionine.
[12] The mutant acetyl CoA hydrolase of [10], wherein the other amino acid is cysteine.
[13] The mutant acetyl CoA hydrolase of any one of [10] to [12], wherein the protein encoded by the acetyl CoA hydrolase gene is a protein as described in (a), (b) or (c) below.
   (a) a protein comprising the amino acid sequence shown in SEQ ID NO: 2;
   (b) a protein comprising an amino acid sequence including a substitution(s), a deletion(s), an insertion(s) and/or an addition(s) of 1 to 10 amino acid residues in the amino acid sequence shown in SEQ ID NO: 2 and having acetyl CoA hydrolase activity;
   (c) a protein comprising an amino acid sequence having 90% or more identity to the amino acid sequence shown in SEQ ID NO: 2 and having acetyl CoA hydrolase activity.
[14] A mutant acetyl CoA hydrolase gene encoding the mutant acetyl CoA hydrolase according to any one of [10] to [13].

### Brief description of the drawings

Figure 1: Accumulation of L-glutamic acid in the wild-type acetyl CoA hydrolase gene-introduced strain and the mutant acetyl CoA hydrolase gene-introduced strain.
Fig. 2: Yield of L-glutamic acid per sugar in the wild-type acetyl CoA hydrolase gene-introduced strain and the mutant acetyl CoA hydrolase gene-introduced strain

### Embodiments for carrying out the invention

The invention is described in detail below.

The method of the present invention is a method of producing L-glutamic acid, comprising: culturing a coryneform bacterium having the L-glutamic acid-producing ability in a culture medium to accumulate L-glutamic acid in the culture medium and/or in cells of the bacterium, and collecting L-glutamic acid from the culture medium and/or the cells, wherein the coryneform bacterium has been modified to harbor a specific genetic mutation. The bacterium used in the method is also referred to as the "bacterium of the present invention.

### <1> Bacterium of the present invention

The bacterium is L-glutamic acid-producing coryneform bacterium that have been modified to harbor the specific genetic mutation.

### <1-1> Coryneform bacterium having L-glutamic acid-producing ability

In the present invention, "coryneform bacterium having L-glutamic acid-producing ability" refers to a coryneform bacterium that have the ability to produce L-glutamic acid and accumulate it in the medium and/or within the bacterial cells to the extent that it can be collected when cultured in the medium. The coryneform bacterium having L-glutamic acid-producing ability may be a coryneform bacterium that is capable of accumulating greater amounts of L-glutamic acid in the medium and/or in the bacterial cells than an unmodified strain. The term "unmodified strain" refers to a control strain that has not been modified to harbor the specific genetic mutation. In other words, the unmodified strain includes the wild strain and the parental strain. The coryneform bacterium having an L-glutamic acid-producing ability may be a coryneform bacterium that is capable of accumulating the target L-amino acid in the medium in an amount of 0.5 g/L or more, preferably 1.0 g/L or more.

The bacterium can produce L-glutamic acid alone or as a mixture of L-glutamic acid and one or more amino acids other than L-glutamic acid, such as L-form amino acids (also called L-amino acids). Examples of L-amino acids include, but not limited to, L-alanine, L-arginine, L-asparagine, L-aspartic acid L-citrulline, L-cysteine, L-glutamic acid, L-glutamine, glycine, L-histidine, L-isoleucine, L-Leucine, L-Lysine, L-Methionine, L-Omithine, L-Phenylalanine, L-Proline, L -serine, L-threonine, L-tryptophan, L-tyrosine, and L-valine.

In the present invention, the terms "glutamic acid" and "amino acid" mean L-glutamic acid and L-amino acid, respectively, unless otherwise noted.

In the present invention, the terms "L-glutamic acid" and "L-amino acid" mean free L-glutamic acid, free L-amino acid, salts thereof, an/or mixtures thereof, unless otherwise noted. Salts are explained below.

The coryneform bacterium includes bacteria belonging to genera such as *Corynebacterium, Brevibacterium* and *Microbacterium.*

Coryneform bacteria specifically include the following species.
*Corynebacterium acetoacidophilum.*
*Corynebacterium acetoglutamicum.*
*Corynebacterium alkanolyticum.*
*Corynebacterium callunae.*
*Corynebacterium crenatum.*
*Corynebacterium glutamicum.*
*Corynebacterium lilium.*
*Corynebacterium melassecola.*
*Corynebacterium thermoaminogenes (Corynebacterium efficiens)*
*Corynebacterium herculis.*
*Brevibacterium divaricatum (Corynebacterium glutamicum)*
*Brevibacterium flavum (Corynebacterium glutamicum)*
*Brevibacterium immariophilum.*
*Brevibacterium lactofermentum (Corynebacterium glutamicum)*
*Brevibacterium roseum.*
*Brevibacterium saccharolyticum.*
*Brevibacterium thiogenitalis*
*Corynebacterium ammoniagenes (Corynebacterium stationis)*
*Brevibacterium album*
*Brevibacterium cerinum.*
*Microbacterium ammoniaphilum.*

*Corynebacterium glutamicum* (formerly known as *Brevibacterium lactofermentum*) is a particular example of a coryneform bacterium.

Coryneform bacteria include, specifically, the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens* (*Corynebacterium thermoaminogenes*) AJ12340 (FERM BP-1539)
*Corynebacterium herculis* ATCC 13868
*Brevibacterium divaricatum* (*Corynebacterium glutamicum*) ATCC 14020
*Brevibacterium flavum* (*Corynebacterium glutamicum*) ATCC 13826, ATCC 14067, AJ12418 (FERM BP-2205)
*Brevibacterium immariophilum* ATCC 14068
*Brevibacterium lactofermentum (Corynebacterium glutamicum)* ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes (Corynebacterium stationis)* ATCC 6871, ATCC 6872
*Brevibacterium album* ATCC 15111
*Brevibacterium cerinum* ATCC 15112
*Microbacterium ammoniaphilum* ATCC 15354

A more particular example of a coryneform bacterium is *Brevibacterium lactofermentum* (updatedname: *Corynebacterium glutamicum*) ATCC 13869.

Another example of a coryneform bacterium is *C. glutamicum* 2256ΔsucAΔldhA yggB* strain, which lacks the ldhA and sucA genes of *Corynebacterium glutamicum* ATCC 13869 and has an IS mutation (V419::IS) in the yggB gene (WO2014/185430).

The bacteria belonging to the genus *Corynebacterium* also includes bacteria that were previously classified in the genus *Brevibacterium* but are now integrated into the genus *Corynebacterium* (Int. J. Syst. Bacteriol., 41, 255(1991)). *Corynebacterium stationis* also includes bacteria that were previously classified as *Corynebacterium ammoniagenes* but were reclassified to *Corynebacterium stationis* based on 16S rRNA sequencing and other analyses (Int. J. Syst. Evol. Microbiol. 60, 874-879 (2010)).

These strains are available, for example, from the American Type Culture Collection, 12301 Parklawn Drive, Rockville, Maryland 20852 P.O. Box 1549, Manassas, VA 20108, United States of America. Each strain is assigned a registration number, which can be used for distribution (http://www.atcc.org/). The registration number corresponding to each strain is listed in the catalog of the American Type Culture Collection. These strains can also be obtained, for example, from the depository where each strain was deposited.

The bacterium of the present invention may be those inherently having L-glutamic acid-producing ability or may be modified to gain L-glutamic acid producing ability. L-glutamic acid-producing bacterium may be obtained by imparting L-glutamic acid producing ability to the bacteria as described above, or by enhancing the L-glutamic acid producing ability of the bacteria as described above.

The imparting or enhancement of L-glutamic acid producing ability can be performed by methods conventionally employed in the breeding of amino acid-producing bacteria such as coryneform bacteria or *Escherichia coli* (Amino Acid Fermentation, Gakkai Publishing Center, Inc. (pages 77 to 100, first published on May 30 1986). Such methods include, for example, obtaining nutrient-auxotrophic mutant strains, obtaining L-glutamic acid analog-resistant strains, obtaining metabolic control mutant strains, and creating recombinant strains with enhanced activity of enzymes in the L-glutamic acid biosynthetic system. Glutamic acid-producing bacterium can be bred for one, two, or three or more of the following characteristics: nutrient auxotrophy, analogue resistance, and metabolic control mutation, etc. The activity of L-glutamic acid biosynthetic enzymes that are enhanced in the breeding of L-glutamic acid-producing bacteria may also be one, two, or three or more. Furthermore, the enhancement of the activity of the biosynthetic enzymes may be combined with the imparting of properties such as nutrient auxotrophy, analog resistance, and metabolic control mutations.

Nutrient-auxotrophic, analog-resistant, or metabolically regulated mutant strains capable of producing L-glutamic acid can be obtained by subjecting the parental or wild type strains to conventional mutagen treatment and selecting from among the mutant strains obtained those that exhibit nutrient-auxotrophy, analog-resistance, or metabolically regulated mutations and also have L-glutamic acid production ability. Conventional mutagen treatments include X-ray or ultraviolet irradiation, N- methyl-N'-nitro-N-nitrosoguanidine (MNNG), ethyl methane sulfonate (EMS), methyl methane sulfonate (MMS), and other mutagen treatments.

The L-glutamic acid producing ability can also be conferred or enhanced by increasing the activity of enzymes involved in the biosynthesis of L-glutamic acid. For example, the enzyme activity can be enhanced by modifying the bacterium so that the expression of the gene encoding the enzyme is increased. Methods for enhancing gene expression are described in WO00/18935, EP1010755A, etc.

L-glutamic acid producing ability can also be imparted or enhanced by reducing the activity of enzymes that catalyze reactions that branch off from the L-glutamic acid biosynthetic pathway to produce compounds other than L-glutamic acid. The term "enzymes that catalyze reactions that branch off from the biosynthetic pathway of L-glutamic acid to produce compounds other than L-glutamic acid" here includes enzymes involved in the degradation of L-glutamic acid.

The following are specific examples of L-glutamic acid producing bacteria and methods of imparting or enhancing L-glutamic acid producing ability. The properties possessed by the L-glutamic acid producing bacteria and the modifications to impart or enhance L-glutamic acid producing ability, as exemplified below, may be used singly or in combination as appropriate.

Methods for imparting or enhancing L-glutamic acid producing ability include, for example, modifying bacteria to increase the activity of one or more enzymes selected from the L-glutamic acid biosynthetic enzymes. Such enzymes include, without limitation, glutamate dehydrogenase (gdhA), glutamine synthetase (glnA), glutamate synthase (gltBD), isocitrate dehydrogenase (icdA), aconitate hydratase (acnA, acnB), citrate synthase (gltA), methylcitrate synthase (prpC), pyruvate carboxylase (pyc), pyruvate dehydrogenase (aceEF, lpdA), pyruvate kinase (pykA, pykF), phosphoenolpyruvate synthase (ppsA), enolase (eno ), phosphoglycerol mutase (pgmA, pgmI), phosphoglycerate kinase (pgk), glyceraldehyde-3-phosphate dehydrogenase (gapA), triosephosphate isomerase (tpiA), fructose bisphosphate aldolase (fbp), glucose phosphate isomerase (pgi), 6-phosphogluconate dehydratase (edd), 2-keto-3-deoxy-6 - phosphogluconate aldolase (edd), and transhydrogenase (pntAB). The names in parentheses are examples of genes encoding the enzymes (the same applies in the following descriptions). Among these enzymes, it is preferred to enhance the activity of one or more enzymes selected from, for example, glutamate dehydrogenase, citrate synthase, phosphoenolpyruvate carboxylase and methylcitrate synthase.

Coryneform bacteria modified to increase expression of the glutamate synthase gene (gltBD) include those disclosed in WO99/07853.

Methods for imparting or enhancing L-glutamic acid producing ability include, for example, modifying bacteria so that the activity of one or more enzymes selected from enzymes that catalyze reactions that branch off from the biosynthetic pathway of L-glutamic acid to produce compounds other than L-glutamic acid is reduced. Such enzymes include, without limitation, isocitrate lyase (aceA), alpha-ketoglutarate dehydrogenase (sucA, odhA), acetolactate synthase (ilvI), formate acetyltransferase (pfl), lactate dehydrogenase ( ldh), alcohol dehydrogenase (adh), glutamic acid decarboxylase (gadAB), and succinate dehydrogenase (sdhABCD). Among these enzymes, it is preferable, for example, to reduce or eliminate alpha-ketoglutarate dehydrogenase activity.

Coryneform bacteria having reduced or deficient α-ketoglutarate dehydrogenase activity and methods for obtaining them are described in WO2008/075483. Specifically, the following strains are exemplified.
*Corynebacterium glutamicum (Brevibacterium lactofermentum)* strain L30-2 (JP2006-340603A)
*Corynebacterium glutamicum (Brevibacterium lactofermentum)* strain ΔS (WO95/34672)
*Corynebacterium glutamicum (Brevibacterium lactofermentum)* AJ12821 (FERM BP-4172; French Patent No. 9401748)
*Corynebacterium glutamicum (Brevibacterium flavum)* AJ12822 (FERM BP-4173; French Patent No. 9401748)
*Corynebacterium glutamicum* AJ12823 (FERM BP-4174; French patent No. 9401748)

L-glutamic acid producing bacteria or parental strains for inducing them also include strains with reduced or deficient in both alpha-ketoglutarate dehydrogenase (sucA) and succinate dehydrogenase (sdh) activities (JP2010-041920A). Such strains specifically include, for example, an odhA, sdhA double-deficient strain of *Corynebacterium glutamicum* ATCC14067 (*Corynebacterium glutamicum* 8L3GΔSDH strain) (JP2010-041920A).

For coryneform bacteria, methods to confer or enhance L-glutamic acid producing ability include methods to confer resistance to organic acid analogs and respiratory inhibitors, as well as methods to confer sensitivity to cell wall synthesis inhibitors. Such methods include, for example, the method of conferring monofluoroacetic acid resistance (JP S50-113209 A), the method of conferring adenine or thymine resistance (JP S57-065198 A), the method of attenuating urease (JP S52-038088 A), the method of conferring malonic acid resistance (JP S52-038088 A), a method for imparting resistance to benzopyrones or naphthoquinones (JP S56-1889 A), a method for imparting HOQNO resistance (JP S56-140895 A), a method for imparting alpha-ketomalonic acid resistance (JP S57-2689 A), guanidine resistance (JP S56-35981 A), and sensitivity to penicillin (JP H4-88994 A).

Examples of such resistant or sensitive bacteria include the following strains.
*Corynebacterium glutamicum (*B*revibacterium flavum)* AJ3949 (FERM BP-2632; JP S50-113209 A)
*Corynebacterium glutamicum* AJ11628 (FERM P-5736; JP S57-065198 A)
*Corynebacterium glutamicum (Brevibacterium flavum)* AJ11355 (FERM P-5007; JP S56-1889 A)
*Corynebacterium glutamicum* AJ11368 (FERM P-5020; JP S56-1889 A)
*Corynebacterium glutamicum (Brevibacterium flavum)* AJ11217 (FERM P-4318; JP S57-2689 A)
*Corynebacterium glutamicum* AJ11218 (FERM P-4319; JP S57-2689 A)
*Corynebacterium glutamicum (Brevibacterium flavum)* AJ11564 (FERM P-5472; JP S56-140895 A)
*Corynebacterium glutamicum (Brevibacterium flavum)* AJ11439 (FERM P-5136; JP S56-35981 A)
*Corynebacterium glutamicum H7684* (FERM BP-3004; JP H04-88994 A)
*Corynebacterium glutamicum (Brevibacterium lactofermentum)* AJ11426 (FERM P-5123; JP H56-48890 A)
*Corynebacterium glutamicum* AJ11440 (FERM P-5137; JP H56-048890 A)
*Corynebacterium glutamicum (Brevibacterium lactofermentum)* AJ11796 (FERM P-6402; JP H58-158192 A)

The activity of L-glutamic acid efflux from bacterial cells can be increased by, for example, increasing the expression of a gene encoding a protein that effluxes L-glutamic acid. The activity to excrete L-glutamic acid can be increased by, for example, increasing the expression of a gene encoding a protein that excretes L-glutamic acid. For example, the b2682 gene (ygaZ), the b2683 gene (ygaH), the b1242 gene (ychE), and the b3434 gene (yhgN) are examples of genes that encode proteins that excrete various amino acids (JP 2002-300874 A).

Another method of imparting or enhancing L-glutamic acid producing ability is, for example, to modify bacteria so that the activity of proteins involved in sugar metabolism or energy metabolism is increased.

Proteins involved in sugar metabolism include proteins involved in sugar uptake and glycolytic enzymes. The genes encoding proteins involved in sugar metabolism include the glucose 6-phosphate isomerase gene (pgi; WO01/02542), pyruvate carboxylase gene (pyc; WO99/18228, EP1092776A), phosphoglucomutase gene (pgm; WO03/04598), fructose diphosphate aldolase gene (pfkB, fbp; WO03/04664), transaldolase gene (talB; WO03/008611), fumarase gene (fum; WO01/02545), non-PTS sucrose uptake gene (csc; EP1149911A), and sucrose utilizing gene (scrAB operon; US Patent No. 7179623).

Genes encoding proteins involved in energy metabolism include the transhydrogenase gene (pntAB; U.S. Patent No. 5830716), cytochrome bo type oxidase gene (cyoB; EP1070376A).

For coryneform bacteria, methods of imparting or enhancing L-glutamic acid producing ability include increasing the expression of the yggB gene or introducing a mutant yggB gene having a mutation in the coding region (WO2006/070944). The bacterium may be modified to increase the expression of the yggB gene or to harbor (have) the mutant yggB gene.

The yggB gene is a gene encoding a mechanosensitive channel. Examples of yggB genes include the yggB gene of corynebacteria. The yggB genes of coryneform bacteria include, for example, the yggB genes from *Corynebacterium glutamicum* ATCC13869, *Corynebacterium glutamicum* ATCC13032, *Corynebacterium glutamicum* ATCC *14967, Corynebacterium melassecola ATCC17965,* and *Corynebacterium callunae* ATCC 15991 (WO2006/070944). The yggB gene from ATCC 13032 strain corresponds to the complementary sequence of the sequence 1,336,091-1,337,692 in the genome sequence registered in the NCBI database under GenBank Accession No. NC_003450, also called NCgl1221. The YggB protein encoded by the yggB gene of *Corynebacterium glutamicum* ATCC13032 is registered as GenBank accession No. NP_600492. The nucleotide sequence of the yggB gene of *Corynebacterium glutamicum* 2256 (ATCC 13869) and the amino acid sequence of the YggB protein encoded by the gene are shown in SEQ ID NO: 7 and SEQ ID NO: 8, respectively.

In the present invention, the yggB gene having the "specific mutation" described below is also referred to as the mutant yggB gene and the protein encoded thereby as the mutant YggB protein. In the present invention, the yggB gene without the "specific mutation" described below is also referred to as the wild-type yggB gene and the protein encoded thereby as the wild-type YggB protein. In the case of YggB protein, the change in the amino acid sequence caused by the "specific mutation" in the yggB gene is also referred to as the "specific mutation. The term "wild type" here is used for convenience to distinguish it from "mutant type," and is not limited to those naturally obtained as long as they do not have such a "specific mutation. The wild-type YggB protein includes the YggB protein exemplified above, for example, a protein having the amino acid sequence shown in SEQ ID NO: 8. The wild-type YggB protein may also include a conserved variant (a variant in which the original function is maintained) of the above example YggB protein that does not have a "specific mutation. The "original function" for YggB protein may be, for example, its function as a mechanosensitive channel, or its ability to enhance L-glutamic acid production in coryneform bacterium when overexpressed in the coryneform bacterium.

Examples of specific mutations are not restricted as long as the mutation alters the amino acid sequence of the wild-type YggB protein as described above and improves the L-glutamic acid producing ability of the coryneform bacterium. The "specific mutation" includes C-terminal mutations and mutations in the transmembrane region (WO2006/070944). The "specific mutation" may also be a combination of such mutations.

### (1) C-terminal side mutation

A C-terminal side mutation is a mutation in the region of the wild-type yggB gene encoding the amino acid residue at position 419 to 533 of the wild-type YggB protein. C-terminal side mutations may be introduced at one or more locations in the region. C-terminal side mutation may cause, for example, substitutions of amino acid residues (missense mutations), insertions of amino acid residues, deletions of amino acid residues, occurrences of stop codons (nonsense mutations), frameshift mutations, or combinations thereof, and among them, insertion of sequences such as insertion sequences (also referred to as "IS") and transposons is preferred.

### (1-1) Sequence insertion

C-terminal side mutations include, for example, mutations that cause the insertion of a sequence at the codon of a valine residue at position 419 of the wild-type YggB protein (2A-1 type mutation). The 2A-1 type mutations can cause, for example, deletion or substitution of some or all of the amino acid residue at 419 to 533 of the wild-type YggB protein. Specifically, a mutant yggB gene having a 2A-1 mutation is, for example, a yggB gene encoding a mutant YggB protein having an IS inserted after the "G" at position 1255 in SEQ ID NO: 7 resulting in a full-length of 423 amino acid residues which is shorter than the original wild-type YggB protein (SEQ ID NO: 8). The nucleotide sequence of this mutant yggB gene (V419::IS) and the amino acid sequence of the mutant YggB protein (V419::IS) encoded by the gene are shown in SEQ ID NO: 9 and SEQ ID NO: 10, respectively. In SEQ ID NO: 9, positions 1-1269 are the CDS of the mutant YggB protein (V419::IS). A specific example of an L-glutamic acid producing bacterium having a mutant yggB gene (V419::IS) is, for example, *C*. *glutamicum* 2256ΔsucAΔldhA yggB* strain (WO2014/185430).

### (1-2) Substitution of proline residue

Examples of C-terminal side mutations include mutations that replace the proline residue in the region of amino acids 419 to 533 of the wild-type YggB protein with another amino acid. Such proline residues include those at positions 424, 437, 453, 457, 462, 469, 484, 489, 497, 515, 529, and 533 of the wild-type YggB protein. Of these, it is preferred to replace the proline residues at positions 424 and/or 437 with another amino acids. The "another amino acids" are for example, but not restricted to, natural-type amino acids other than proline. The another amino acid includes Lys, Glu, Thr, Val, Leu, Ile, Ser, Asp, Asn, Gln, Arg, Cys, Met, Phe, Trp, Tyr, Gly, Ala, His. For example, the proline residue at position 424 may be replaced preferably with a hydrophobic amino acid (Ala, Gly, Val, Leu, or Ile) and more preferably with a branched-chain amino acid (Leu, Val, or Ile). For example, the proline residue at position 437 may be replaced with an amino acid preferably having a hydroxyl group on the side chain (Thr, Ser, or Tyr), and more preferably with Ser.

### (2) Mutation in the transmembrane regions

The YggB protein is considered to have five transmembrane regions. The transmembrane regions correspond to amino acid residues at 1 to 23 (first transmembrane region), 25 to 47 (second transmembrane region), 62 to 84 (third transmembrane region), 86 to 108 (fourth transmembrane region), and 110 to 132 (fifth transmembrane region) of the wild-type YggB protein, respectively. Mutations in the transmembrane regions are mutations in the regions encoding these transmembrane regions in the wild-type yggB gene. Mutations in the transmembrane regions may be introduced at one or more locations in the same region. Mutations in the transmembrane region may be one that causes one or several amino acid substitutions, deletions, additions, insertions, or inversions, but does not cause frameshift mutation or nonsense mutation. "One or several" means preferably from 1 to 20, more preferably from 1 to 10, more preferably from 1 to 5, and especially preferably from 1 to 3. Mutations in the transmembrane region include the insertion of one or several amino acids (e.g., Cys-Ser-Leu) between the leucine residue at position 14 and the tryptophan residue at position 15 of the wild-type YggB protein, substitution of the alanine residue at position 100 with another amino acid residue (e.g., an amino acid with a hydroxyl group in its side chain, such as Thr, Ser, or Tyr, preferably Thr), and substitution of the alanine residue at position 111 with another amino acid residue (e.g., Val or an amino acid with a hydroxyl group in its side chain, such as Thr, Ser, or Tyr, preferably Val or Thr).

In the present invention, "amino acid residue at position X of wild-type YggB protein" means an amino acid residue corresponding to the amino acid residue at position X in SEQ ID NO: 8, unless otherwise specified. The "X position" in the amino acid sequence means the Xth position counting from the N-terminal of the amino acid sequence, where the N-terminal amino acid residue is the amino acid residue at position 1. The position of an amino acid residue indicates its relative position, and its absolute position may change due to deletion, insertion, or addition of amino acids. For example, "amino acid residue at position 419 of wild-type YggB protein" means an amino acid residue corresponding to the amino acid residue at position 419 in SEQ ID NO: 8, and if one amino acid residue on the N-terminal side is deleted from position 419, the amino acid residue 418th from the N-terminus shall be "the amino acid residue at position 419 of wild-type YggB protein". If one amino acid residue is inserted on the N-terminal side of the protein, the amino acid residue 420th from the N-terminus shall be the "amino acid residue at position 419 of the wild-type YggB protein". Specifically, for example, in the YggB protein of *Corynebacterium glutamicum* ATCC14967 strain, the amino acid residue at position 419-529 corresponds to the amino acid residue at position 419-533 of the wild-type YggB protein. Also, for example, the alanine residue at position 98 in the YggB protein of *Corynebacterium callunae* corresponds to the alanine residue at position 100 in the wild-type YggB protein.

In the amino acid sequence of any YggB protein, which amino acid residue is "the amino acid residue corresponding to the amino acid residue at position X in SEQ ID NO: 8" can be determined by aligning the amino acid sequence of the YggB protein with that of SEQ ID NO: 8. The alignment can be performed, for example, by using known gene analysis software. Specific software includes DNASIS by Hitachi Solutions and GENETYX by Xenetics (Elizabeth C. Tyler et al., Computers and Biomedical Research, 24(1), 72-96, 1991; Barton GJ et al., Journal of molecular biology, 198(2), 327-37. 1987).

The mutant yggB gene can be obtained by modifying the wild-type yggB gene to have the "specific mutation" described above. Specifically, for example, a site-directed mutation method to introduce the desired mutation at the target site of DNA can be achieved by using PCR (Higuchi, R., 61, in PCR technology, Erlich, H. A. Eds., Stockton press (1989); Carter, P.,. Meth. In Enzymol., 154, 382 (1987)) and methods using phage (Kramer, W. and Frits, H. J., Meth. In Enzymol., 154, 350 (1987); Kunkel, T. A. et al. 154, 367 (1987)). The mutant yggB gene can also be obtained by chemical synthesis.

Modifying a coryneform bacterium to have a mutant yggB gene can be achieved by introducing a mutant yggB gene into the coryneform bacterium. Modification of the coryneform bacteria to have the mutant yggB gene can also be achieved by introducing mutations into the yggB gene possessed by the bacterium through spontaneous mutation or mutagen treatment.

### <1-2> Modification in the mutant acetyl CoA hydrolase gene

The coryneform bacterium have been modified to harbor a mutant acetyl CoA hydrolase gene encoding a mutant acetyl CoA hydrolase having the substitution of a serine residue at position 383 in the amino acid sequence of wild-type acetyl CoA hydrolase with another amino acid residue.

The coryneform bacterium of the present invention may be one modified to harbor the mutant acetyl CoA hydrolase gene in a coryneform bacterium having L-glutamic acid production ability as described above, or may be a coryneform bacterium modified to harbor the mutant acetyl CoA hydrolase gene and then imparting the L-glutamic acid producing ability. Also included are coryneform bacteria that have been modified to harbor the mutant acetyl CoA hydrolase gene thereby gaining the L-glutamic acid producing ability.

The acetyl-CoA hydrolase gene is a gene that encodes acetyl-CoA hydrolase. The acetyl-CoA hydrolase gene may be an acetyl-CoA hydrolase gene from coryneform bacteria. The acetyl CoA hydrolase genes from coryneform bacteria include, for example, acetyl CoA hydrolase genes from *Corynebacterium glutamicum* ATCC13869, *Corynebacterium glutamicum* ATCC13032, *Corynebacterium* ATCC14967 and *Corynebacterium melassecola* ATCC17965. The acetyl CoA hydrolase gene from *Corynebacterium glutamicum* ATCC13032 corresponds to the complementary sequence of 2,729,376 to 2,730,884 in the genome sequence deposited in the NCBI database under GenBank Accession No. NC_003450, also called NCgl2480. The acetyl CoA hydrolase encoded by the acetyl CoA hydrolase gene from ATCC13032 has been registered as GenBank accession No. WP_003858947.1. The nucleotide sequence of the wild-type acetyl CoA hydrolase gene from *Corynebacterium glutamicum* 2256 (ATCC 13869) and the amino acid sequence of the acetyl CoA hydrolase encoded by the gene are shown in SEQ ID NO: 1 and SEQ ID NO: 2, respectively.

Acetyl-CoA hydrolase" may refer to a protein that has the activity to catalyze the hydrolysis of acetyl-CoA to produce coenzyme A and acetic acid and/or the reverse reaction (e.g., EC 3.1.2.1). The activity is also referred to as "acetyl CoA hydrolase activity. Acetyl CoA hydrolase activity may specifically be the activity that catalyzes the reaction that converts acetyl CoA and H₂O to coenzyme A and acetic acid and/or the reverse reaction. Acetyl-CoA hydrolase is also referred to as "acetyl-CoA deacylase". An example of the gene encoding acetyl-CoA hydrolase includes the NCgl2480 gene. The nucleotide sequences of acetyl-CoA hydrolase genes such as NCgl2480 gene possessed by the bacterium to be modified and the amino acid sequences of the acetyl-CoA hydrolases encoded by them can be obtained from public databases, for example, NCBI.

In the present invention, an acetyl CoA hydrolase gene having the "specific genetic mutation" as described below is also referred to as a mutant acetyl CoA hydrolase gene and the protein encoded thereby as mutant acetyl CoA hydrolase. In the present invention, an acetyl CoA hydrolase gene that does not have the "specific genetic mutation" described below is also referred to as a wild-type acetyl CoA hydrolase gene and the protein encoded thereby as wild-type acetyl CoA hydrolase. In the case of acetyl CoA hydrolase, a change in the amino acid sequence caused by a "specific genetic mutation" in the acetyl CoA hydrolase gene is also referred to as a "specific mutation. The term "wild type" here is used for convenience to distinguish it from "mutant type," and is not limited to those obtained naturally as long as they do not have the "specific genetic mutation. The wild-type acetyl CoA hydrolase includes the acetyl CoA hydrolase exemplified above, for example, a protein comprising the amino acid sequence shown in SEQ ID NO: 2. Also included in the wild-type acetyl CoA hydrolase are conservative variants (variants in which the original function is maintained) of the above exemplified acetyl CoA hydrolases, which do not have the "specific genetic mutations".

The "specific genetic mutation" is a mutation that alters the amino acid sequence of wild-type acetyl CoA hydrolase, as described above, to improve the L-glutamic acid producing ability of the coryneform bacterium. Specifically, it is a mutation in the amino acid residue at position 383 of the amino acid sequence of wild-type acetyl CoA hydrolase in the wild-type acetyl CoA hydrolase gene.

The mutation in the amino acid residue at position 383 in the amino acid sequence of wild-type acetyl CoA hydrolase include, for example, substitutions of amino acid residue (missense mutation).

For example, a mutation in the amino acid residue at position 383 of wild-type acetyl CoAhydrolase is a mutation in which the serine residue at position 383 of wild-type acetyl CoA hydrolase is replaced by another amino acid residue. The "another amino acid" is not restricted as long as it is any naturally occurring amino acid other than serine. Another amino acid includes Lys, Glu, Thr, Gly, Val, Leu, Ile, Asp, Asn, Gln, Arg, Cys, Met, Phe, Trp, Tyr, Pro, Ala, His. For example, the serine residue at position 383 may be replaced by a hydrophilic amino acid (Lys, Glu, Thr, Asp, Asn, Gln, Arg, Cys or His), preferably by Cys. For example, the serine residue at position 383 may be replaced by a sulfur-containing amino acid (Cys or Met), preferably by Cys.

In the present invention, "amino acid residue at position X of wild-type acetyl CoA hydrolase" means an amino acid residue corresponding to the amino acid residue at position X in SEQ ID NO: 2, unless otherwise specified. Position X" in the amino acid sequence means the Xth position counted from the N-terminal of the same amino acid sequence, where the N-terminal amino acid residue is the amino acid residue at position 1. The position of an amino acid residue indicates its relative position, and its absolute position may change due to deletion, insertion, or addition of amino acids. For example, "amino acid residue at position 383 of wild-type acetyl CoA hydrolase" means the amino acid residue corresponding to position 383 in SEQ ID NO: 2. If one amino acid residue on the N-terminal side is deleted, the 382^{nd} amino acid residue from the N-terminus shall be "the amino acid residue at position 383 of wild-type acetyl CoA hydrolase. If one amino acid residue is inserted on the N-terminal side of the protein, the 384^{th} amino acid residue from the N-terminus shall be the "amino acid residue at position 383 of the wild-type acetyl CoA hydrolase".

In the amino acid sequence of any acetyl CoA hydrolase, which amino acid residue is "the amino acid residue corresponding to the amino acid residue at position X in SEQ ID NO: 2" can be determined by aligning the amino acid sequence of the acetyl CoA hydrolase with the amino acid sequence of SEQ ID NO: 2. Alignment can be performed, for example, by using known genetic analysis software. Specific software includes DNASIS by Hitachi Solutions and GENETYX by Xenetics (Elizabeth C. Tyler et al., Computers and Biomedical Research, 24(1), 72-96, 1991; Barton GJ et al., Journal of molecular biology, 198(2), 327-37. 1987).

The mutant acetyl CoA hydrolase gene can be obtained by modifying the wild-type acetyl CoA hydrolase gene to have the "specific genetic mutation" described above. Specifically, for example, a site-directed mutation method to introduce the target mutation at the target site of DNA is a method using PCR (Higuchi, R., 61, in PCR technology, Erlich, H. A. Eds., Stockton press (1989); Carter, P., et al. Meth. In Enzymol., 154, 382 (1987)) or using phage (Kramer, W. and Frits, H. J., Meth. In Enzymol., 154, 350 (1987); Kunkel, T. A. et al. 154, 367 (1987)). Mutant acetyl CoA hydrolase gene can also be obtained by chemical synthesis.

The wild-type acetyl CoA hydrolase gene may be, for example, a gene having the nucleotide sequence of the above-exemplified acetyl CoA hydrolase gene (e.g., the nucleotide sequence shown in SEQ ID NO: 1). The acetyl CoA hydrolase may be, for example, a protein comprising the amino acid sequence of the above acetyl CoA hydrolase (e.g., the amino acid sequence shown in SEQ ID NO: 2). The expression "having the (amino acid or nucleotide) sequence" means "comprising the (amino acid or nucleotide) sequence" and includes the case "consisting of the (amino acid or nucleotide) sequence" unless otherwise specified.

The wild-type acetyl CoA hydrolase gene may also be a variant of the above-exemplified acetyl CoA hydrolase gene (e.g., a gene having the sequence shown in SEQ ID NO: 1) as long as the original function is maintained. Similarly, the acetyl CoA hydrolase may be a variant of the above example acetyl CoA hydrolase (e.g., a protein having the amino acid sequence shown in SEQ ID NO: 2) as long as the original function is maintained. Such a variant in which the original function is maintained may be referred to as a "conservative variant. The term "acetyl CoA hydrolase gene" shall encompass such conserved variants as well as the above exemplified acetyl CoA hydrolase genes. Similarly, the term "acetyl CoA hydrolase" shall encompass such conserved variants as well as the above exemplified acetyl CoA hydrolases. Conservative variants include, for example, homologs or artificial variants of the above exemplified acetyl CoA hydrolase genes and acetyl CoA hydrolases.

. The term "original function is maintained" means that the gene or protein variant has a function (e.g., activity or property) that corresponds to the function (e.g., activity or property) of the original gene or protein. The "original function is maintained" for a gene means that the gene variant encodes a protein for which the original function is maintained. In other words, "original function is maintained" for the acetyl CoAhydrolase gene may mean that the variant of the acetyl CoA hydrolase gene encodes a protein that has acetyl CoA hydrolase activity. Also, "original function is maintained" for acetyl CoA hydrolase may mean that the variant of acetyl CoA hydrolase has acetyl CoA hydrola ase activity.

Acetyl CoA hydrolase activity can be measured, for example, by incubating the enzyme with the corresponding substrate (e.g., acetyl CoA and H₂O) and measuring enzyme- and substrate-dependent production of the corresponding products (e.g., coenzyme A and acetic acid).

The following are examples of conservative variants.

Homologs of the acetyl CoA hydrolase gene or homologs of the acetyl CoA hydrolase can be easily obtained from public databases, for example, by BLAST or FASTA searches using the nucleotide sequence of the above acetyl CoA hydrolase gene or the amino acid sequence of the above acetyl CoA hydrolase as the query sequence. Homologs of the acetyl CoA hydrolase gene can also be obtained, for example, by PCR using the chromosomes of various organisms as templates and oligonucleotides made based on these known acetyl CoA hydrolase gene sequences as primers.

The acetyl CoA hydrolase gene may be a gene encoding a acetyl CoA hydrolase having an amino acid sequence in which one or several amino acids at one or several positions are substituted, deleted, inserted and/or added in the above amino acid sequence (e.g., amino acid sequence shown in SEQ ID NO: 2), as long as the original function is maintained. For example, the encoded protein may have its N- and/or C-terminus extended or shortened. The above "one or several" means, depending on the position and type of amino acid residues in the three-dimensional structure of the protein, specifically, for example, 1 to 50, 1 to 40, 1 to 30, preferably 1 to 20, more preferably 1 to 10, more preferably 1 to 5, and especially preferably 1 to 3.

The above substitutions, deletions, insertions and/or additions of one or several amino acids are conservative mutations in which the function of the protein is normally maintained. A typical conservative mutation is a conservative substitution. Conservative substitutions are between Phe, Trp, and Tyr when an aromatic amino acid is substituted, between Leu, Ile, and Val when a hydrophobic amino acid is substituted, between Gln and Asn when a polar amino acid is substituted, and between Lys, Arg, and His when a basic amino acid is substituted, Asp and Glu when an acidic amino acid is substituted, and between Ser and Thr when amino acid having a hydroxyl group is substituted. Substitutions that are considered as conservative substitutions are, specifically, substitutions from Ala to Ser or Thr, from Arg to Gln, His or Lys, from Asn to Glu, Gln, Lys, His or Asp, from Asp to Asn, Glu or Gln, from Cys to Ser or Ala; from Gln to Asn, Glu, Lys, His, Asp or Arg; from Glu to Gly, Asn, Gln, Lys or Asp; from Gly to Pro; from His to Asn, Lys, Gln, Arg or Tyr; from Ile to Leu, Met, Val or Phe; from Leu to Ile, Met, Val or Phe; from Lys to Asn, Glu, Gln, His or Arg; from Met to Ile, Leu, Val or Phe; from Phe to Trp, Tyr, Met, Ile or Leu; from Ser to Thr or Ala, from Thr to Ser or Al; from Trp to Phe or Tyr, from Tyr to His, Phe or Trp, and from Val to Met, Ile or Leu. The above substitutions, deletions, insertions, or additions of amino acids also include those caused by naturally occurring mutations (mutant or variant), such as based on individual differences or species differences of the organism from which the gene is derived.

The acetyl CoA hydrolase gene may also be a gene encoding a protein comprising an amino acid sequence having, for example, 50% or more, 65% or more, 80% or more, preferably 90% or more, more preferably 95% or more, more preferably 97% or more, especially more preferably 99% or more sequence identity to the entire amino acid sequence, as long as the original function is maintained.

The acetyl CoA hydrolase gene may also be a gene (e.g., DNA) that hybridizes under stringent conditions with a probe that can be prepared from the above sequence (e.g., the sequence shown in SEQ ID NO: 1), such as a complementary sequence to the whole or part of the above sequence, as long as the original function is maintained. The term "under stringent conditions" means under the following conditions. Stringent conditions" refers to conditions under which so-called specific hybrids are formed and nonspecific hybrids are not formed. One example is a condition under which DNAs of high identity hybridize with each other, e.g., at least 50%, at least 65%, at least 80%, preferably at least 90%, more preferably at least 95%, even more preferably at least 97%, especially more preferably at least 99%, and DNAs of lower identity do not hybridize with each other. Examples of such conditions include the conditions of normal Southern hybridization in which washing at a salt concentration and temperature corresponding to 60°C, 1× SSC, 0.1% SDS, preferably 60°C, 0.1× SSC, 0.1% SDS, more preferably 68°C, 0.1× SSC, 0.1% SDS is performed once, preferably 2-3 times can be mentioned.

As mentioned above, the probe used for the above hybridization may be part of the complementary sequence of the gene. Such probes can be made by PCR using oligonucleotides made based on known gene sequences as primers and DNA fragments containing the gene described above as templates. For example, a DNA fragment of about 300 bp in length can be used as a probe. When a DNA fragment of about 300 bp in length is used as a probe, the conditions for hybridization washing include 50°C, 2× SSC, and 0.1% SDS.

The acetyl CoA hydrolase gene may also have any codon change with its equivalent codon, since the codon degeneration occurs from host to host. In other words, the acetyl CoA hydrolase gene may be a variant of the acetyl CoA hydrolase gene exemplified above due to genetic code degeneration. For example, the acetyl CoA hydrolase gene may be modified to have the optimal codon usage according to the codon usage frequency of the host used.

The "identity" between amino acid sequences means the identity between amino acid sequences calculated by blastp using the default settings of Scoring Parameters (Matrix: BLOSUM62; Gap Costs: Existence=11, Extension=1; Compositional Adjustments: Conditional compositional score matrix adjustment). The "identity" between nucleotide sequences means the identity between nucleotide sequences calculated by blastn using the default settings of Scoring Parameters (Match/Mismatch Scores=1,-2; Gap Costs=Linear).

The above description of conservative variants of genes and proteins can be applied mutatis mutandis to any protein and the genes encoding them. That is, genes and proteins used for breeding L-glutamic acid producing bacteria may, for example, have the nucleotide and amino acid sequences of known genes and proteins, such as the genes and proteins exemplified above, respectively. The genes and proteins used for breeding L-glutamic acid producing bacteria may also be conservative variants of known genes and proteins, such as the genes and proteins exemplified above, respectively. Specifically, for example, a gene used for breeding L-glutamic acid producing bacteria may be a gene encoding a protein having an amino acid sequence in which one or several amino acids at one or several positions are substituted, deleted, inserted or added in the amino acid sequence of a known protein, as long as the original function is maintained.

The method of modifying the bacterium to harbor the mutant acetyl CoA hydrolase gene includes the method of introducing a mutant acetyl CoA hydrolase gene in which the serine residue at position 383 is replaced within the coding region of the wild-type acetyl CoA hydrolase gene into a coryneform bacterium, the method of introducing the same mutation into the coding region of the wild-type acetyl CoA hydrolase gene that the coryneform bacterium possesses, and so on.

Modification of the coryneform bacterium to have a mutant acetyl CoA hydrolase gene can be achieved by introducing a mutant acetyl CoA hydrolase gene into the coryneform bacterium. Modification of the coryneform bacterium to have the mutant acetyl CoA hydrolase gene can also be achieved by introducing mutations into the acetyl CoA hydrolase gene of the coryneform bacteria by spontaneous mutation or mutagen treatment.

The introduction of a mutant acetyl CoA hydrolase gene into a coryneform bacterium can be accomplished by introducing the gene into the host chromosome. Introduction of the gene into the chromosome can be accomplished, for example, by homologous recombination (Miller, J. H. Experiments in Molecular Genetics, 1972, Cold Spring Harbor Laboratory). Gene transfer methods using homologous recombination include, for example, the red-driven integration method (Datsenko, K. A., and Wanner, B. L. Proc. Natl. Acad. Sci. U S A. 97:6640-6645 ( U S A. 97:6640-645 ( 2000)), plasmids containing a temperature-sensitive replication origin, plasmids capable of junctional transmission, suicide vectors without a replication origin that functions within the host, and phage-based transduction methods are examples. Specifically, the gene can be introduced onto the host chromosome by transforming the host with recombinant DNA containing the mutant acetyl CoA hydrolase gene and causing homologous recombination with the target site on the host chromosome. The structure of the recombinant DNA used for homologous recombination is not restricted as long as homologous recombination occurs in the desired manner. For example, a linear DNA containing a mutant acetyl CoA hydrolase gene and having homologous sequences upstream and downstream of the target site on the chromosome at both ends of the gene can be used to transform the host and cause homologous recombination upstream and downstream of the target site, respectively, to replace the target site with the gene. The recombinant DNA used for homologous recombination may be equipped with a marker gene for selecting transformants. Only one copy of the gene may be introduced, or two or more copies may be introduced. For example, a large number of copies of a mutant acetyl CoA hydrolase gene can be introduced into a chromosome by homologous recombination targeting a sequence with a large number of copies on the chromosome. Sequences with many copies on the chromosome include repetitive DNA sequences (repetitive DNA) and inverted repeats at both ends of the transposon. Homologous recombination may also be performed by targeting appropriate sequences on the chromosome, such as genes that are not required for the production of the target substance. Genes can also be introduced randomly on the chromosome using transposons or Mini-Mu (JP H2-109985 A, U.S. Patent No. 5882888, EP805867B1). This method of modifying chromosomes using homologous recombination is not limited to the introduction of mutant acetyl CoA hydrolase genes, but can be used for any modification of chromosomes, such as modification of expression regulatory sequences.

Confirmation of the introduction of the mutant acetyl CoA hydrolase gene on the chromosome can be confirmed by Southern hybridization using a probe with a sequence complementary to all or part of the gene, or by PCR using primers based on the sequence of the gene.

Introduction of the mutant acetyl CoA hydrolase gene into coryneform bacterium can also be achieved by introducing a vector containing the gene into the host. For example, a DNA fragment containing the mutant acetyl CoA hydrolase gene can be linked to a vector that functions in the host to construct an expression vector for the gene, and the gene can be introduced into the coryneform bacterium by transforming the host with the expression vector. DNA fragments containing the mutant acetyl CoA hydrolase gene can be obtained, for example, by PCR using genomic DNA of a microorganism having the mutant acetyl CoA hydrolase gene as a template. As a vector, a vector capable of autonomous replication in the host cell can be used. The vector may preferably be a multi-copy vector. In order to select transformants, the vector may have a marker such as an antibiotic resistance gene. The vector may also have a promoter or terminator for expression of the inserted gene. Vectors may be, for example, vectors derived from bacterial plasmids, yeast plasmids, bacteriophage vectors, cosmids, or phage mids. Vectors capable of autonomous replication in coryneform bacteria include, for example, pHM1519 (Agric. Biol. Chem., 48, 2901-2903 (1984)); pAM330 (Agric. Biol. Chem., 48, 2901-2903 (1984)); modified versions of these plasmids with drug resistance genes; pCRY30 (JP H3-210184 A); pCRY21, pCRY2KE, pCRY2KX, pCRY31, pCRY3KE, and pCRY3KX (JP H2-72876 A, US Patent 5185262); pCRY2 and pCRY3 (JP H01-191686A); pAJ655, pAJ611 and pAJ1844 (JP S58-192900A); pCG1 (JP S57-134500A); pCG2 (JP S58-35197A); pCG4 and pCG11 (JP S57-183799A); pVK7 (JP H10-215883 A); pVK9 (US Patent Application Publication No. 2006/0141588); pVC7 (JP H9-070291 A); pVS7 (WO2013/069634).

When introducing a gene, the gene need only be harbored in the host to be expressible. Specifically, the gene may be harbored so that it can be expressed under the control of a promoter that functions in the host. The promoter is not limited as long as it can function in the host. A "promoter that functions in the host" refers to a promoter that has promoter activity in the host. The promoter may be a promoter of host origin or a promoter of heterologous origin. The promoter may be a specific promoter of the gene to be introduced, or it may be a promoter of another gene.

A terminator for transcription termination can be placed downstream of the gene. Terminators are not restricted as long as they are functional in the host. The terminator can be a host-derived terminator or a terminator of heterologous origin. The terminator may be a unique terminator of the gene to be transduced, or it may be a terminator of another gene.

Vectors, promoters, and terminators that can be used in various microorganisms are described in detail in, for example, "Basic Microbiology Course 8: Gene Engineering," Kyoritsu Shuppan, 1987.

When introducing two or more genes, each gene may be harbored in the host in an expressible manner. For example, each gene may be held on a single expression vector, or all may be held on a chromosome. Each gene may be held separately on multiple expression vectors, and may be held separately on a single or multiple expression vectors and on the chromosome. The operon may also be composed of two or more genes and introduced. The "case of introducing two or more genes" includes, for example, the introduction of genes encoding two or more proteins (e.g., enzymes), genes encoding two or more subunits of a single protein complex (e.g., an enzyme complex), and combinations thereof.

### <2>Production method of L-glutamic acid

The method of the present invention is a method for producing L-glutamic acid, comprising culturing the bacterium of the invention described in <1> above in a culture medium to accumulate L-glutamic acid in the culture medium and/or in the cells of the bacterium, and collecting L-glutamic acid from the culture medium and/or the cells. L-glutamic acid is as described above. In the present invention, L-glutamic acid may be produced alone, or L-glutamic acid and one or more amino acids other than L-glutamic acid, such as L-amino acids (L-amino acids), etc., may be produced.

The medium used is not restricted as long as the bacteria can grow and produce L-glutamic acid. For example, ordinary culture media used for culturing bacteria such as coryneform bacteria can be used. For example, a medium containing a carbon source, nitrogen source, phosphate source, sulfur source, and other components selected from various organic and inorganic components as needed can be used. The type and concentration of the medium components may be set according to various conditions, such as the type of bacterium to be used.

As carbon sources, specifically, sugars such as glucose, fructose, sucrose, lactose, galactose, xylose, arabinose, maltose, isomaltose, waste molasses, starch hydrolysates, biomass hydrolysates, organic acids such as acetic acid, fumaric acid, citric acid, succinic acid, alcohols such as glycerol, crude glycerol, ethanol, and fatty acids can be used. Carbon sources include, in particular, sugars. More particularly, glucose and fructose are mentioned as carbon sources. Sugars such as glucose and fructose may be used as carbon sources alone or in combination with other carbon sources. For example, a sugar with fructose as a constituent sugar may be used as a carbon source. Fructose, sucrose, and fructooligosaccharides are examples of fructose-based sugars. Fructose-based sugars may be used as a carbon source alone or in combination with other carbon sources. Plant-derived materials can be used as carbon sources. Examples of plants include corn, rice, wheat, soybeans, sugarcane, beets, and cotton. Plant-derived raw materials include, for example, organs such as roots, stems, trunks, branches, leaves, flowers, and seeds, plant bodies including these organs, and decomposition products of these plant organs. Plant-derived raw materials can be used in any form, such as unprocessed products, squeezed juice, crushed products, and refined products. As a carbon source, for example, kain-molasses, beet-molasses, high-test-molasses, citrus-molasses, or invert sugar may be used, or hydrolyzed products of natural raw materials such as cellulose, starch, corn, cereal, tapioca, and cassava may be used. Pentasaccharides such as xylose, hexasaccharides such as glucose, or mixtures thereof can be obtained from plant biomass, for example. Specifically, these sugars can be obtained by subjecting plant biomass to treatment such as steam treatment, concentrated acid hydrolysis, dilute acid hydrolysis, hydrolysis by enzymes such as cellulase, and alkali treatment. Since hemicellulose is generally more easily hydrolyzed than cellulose, hemicellulose in plant biomass can be hydrolyzed beforehand to release pentose carbohydrates, and then cellulose can be hydrolyzed to produce hexoses. Xylose may also be provided by conversion from hexa-carbohydrates, for example, by having the bacterium possess a conversion pathway from hexa-carbohydrates such as glucose to xylose. The carbon source may be, for example, glucose alone or a mixture of two carbon sources such as glucose and fructose or glucose and sucrose in any ratio (e.g., 3:7 to 7:3 by weight).

As nitrogen sources, specifically, organic nitrogen sources such as ammonium salts such as ammonium sulfate, ammonium chloride, ammonium phosphate, etc., peptone, yeast extract, meat extract, plant protein hydrolysate (HVP; e.g., soy protein hydrolysate, soy sauce, pea soy sauce, etc.), ammonia, ammonia gas or ammonia water used for pH adjustment may be used as a nitrogen source. One nitrogen source may be used, or a combination of two or more nitrogen sources may be used.

Specific examples of phosphoric acid sources include phosphates such as potassium dihydrogen phosphate, potassium dihydrogen phosphate, and phosphate polymers such as pyrophosphoric acid. One phosphoric acid source may be used, or a combination of two or more phosphoric acid sources may be used.

Specific examples of sulfur sources include inorganic sulfur compounds such as sulfates, thiosulfates, sulfites, and sulfur-containing amino acids such as cysteine, cystine, and glutathione. One sulfur source may be used, or a combination of two or more sulfur sources may be used.

Various other organic and inorganic components, specifically, inorganic salts such as sodium chloride, potassium chloride, etc.; trace metals such as iron, manganese, magnesium, calcium, etc.; vitamins such as vitamins B1, vitamin B2, vitamin B6, nicotinic acid, nicotinic acid amide, vitamin B12 , biotin, folic acid, etc.; amino acids; nucleic acids; organic ingredients such as peptone, casamino acids, yeast extract, plant protein hydrolysates (HVP; e.g., soy protein hydrolysates, soy sauce, pea soy sauce, etc.) that contain these ingredients. Other various organic and inorganic ingredients include defoamers, osmotic pressure regulators of the culture medium, osmotic pressure compensating substances, etc. Antifoamers include silicone antifoamers (oil, solution, oil compound, emulsion, self-emulsifying, etc.), alcohol-based antifoamers, oil-based antifoamers, polyether-based antifoamers, vegetable oils (cottonseed oil, linseed oil, soybean oil, olive oil, castor oil, coconut oil, etc.). Antifoamers can be used in any form, such as liquid, paste, solid, powder, emulsion, wax, etc. Osmotic compensating substances for the culture medium include salts such as sodium chloride and potassium chloride, and polysaccharides (sorbitol, dextrin, etc.) that microorganisms cannot utilize. Osmotic compensating substances include potassium ions, betaine (glycine betaine), waste molasses (especially sugar beet waste molasses), glutamic acid, trehalose, etc. Polymers selected from the group consisting of water-soluble cellulose derivatives, water-soluble polyvinyl compounds, polar organic solvent soluble polyvinyl compounds, water-soluble starch derivatives, alginates, and polyacrylates are also acceptable components to be added to the culture medium. These various other organic and inorganic components may be used as a single component or in combination with two or more components.

If a nutrient-auxotrophic mutant strain that requires amino acids or other nutrients for growth is used, it is preferable to supplement the medium with the required nutrients.

Limiting the amount of biotin in the medium and adding surfactants or penicillin to the medium are also preferred.

Culture conditions are not restricted as long as the bacterium can grow and produce L-glutamic acid. Culturing can be performed under the conventional conditions used for culturing bacteria such as coryneform bacteria. The culture conditions may be set appropriately according to various conditions, such as the type of bacterium used.

Culture can be performed using liquid medium. For the method of liquid culture, it is possible to use, for example, the method described in "Biotechnology Textbook Series 13: Culture Engineering, Toshiomi Yoshida, Corona, 1998. For liquid culture, it is possible to use, for example, surface culture, deep culture, membrane (such as dialysis membrane or hollow-fiber separated culture, or immobilized microbial culture. In addition, culture equipment can be used, for example, aerated agitation type culture equipment, airlift type culture equipment, packed-bed type culture equipment, or fluidized-bed type culture equipment. For the culture, it is possible to use the method described in "Fundamentals of Fermentation Engineering, Gakkai Shuppan Center, 1988". During culture, the bacterium cultured in solid medium such as agar medium may be directly inoculated into liquid medium, or the bacterium may be seed cultured in liquid medium and inoculated into liquid medium for the main culture. In other words, the culture may be divided into seed culture and main culture. In such cases, the culture conditions for the seed culture and main culture may or may not be the same. The amount of the bacterium contained in the culture medium at the start of the culture is not restricted. The main culture may be performed, for example, by inoculating 1 to 50% (v/v) of the seed culture medium into the main culture medium. For example, the seed culture process may include two or more stages of seed culture to obtain the amount of bacterial cells required for the main culture process. The seed culture medium may be inoculated only at the start of the main culture, or may be inoculated at the start of the main culture and additionally during the main culture.

Culture can be conducted in batch culture, fed-batch culture, continuous culture, or a combination thereof. For example, two or more stages of fed-batch culture or two or more stages of continuous culture can be used as a combination. The medium at the start of culture is also referred to as "initial culture medium. The medium supplied to the culture system (fermenter) in a fed-batch culture or continuous culture is also called "feed medium. Also, supplying feed medium to a culture system in a fed-batch culture or continuous culture is also referred to as "feed". When the culture is divided into seed culture and main culture, for example, both the seed culture and the main culture may be performed in a batch culture. For example, the seed culture may be done in batch culture, and the main culture may be done in fed-batch culture or continuous culture. For example, the seed culture may be performed in a fed-batch culture and the main culture may be performed in a batch culture. The culture medium may be supplied from a point in the top of the culture tank that is not in contact with the culture medium surface, from a point inside the culture medium such as the middle of the culture tank or the bottom of the culture tank, or from the top and middle of the culture tank, respectively. The method of supplying the feed medium from a point inside the culture medium is disclosed, for example, in JP6097869B.

In the present invention, each culture medium component may be contained in the primary culture medium, the feed medium, or both. The type of component contained in the initial culture medium may or may not be the same as the type of component contained in the feed medium. The concentration of each component contained in the initial medium may or may not be the same as the concentration of each component contained in the feed medium. Two or more feed mediums with different types and/or concentrations of the contained components may be used. For example, if multiple rounds of feeding are performed intermittently, the type and/or concentration of components contained in each round of feed medium may or may not be the same. For example, the carbon source of the initial medium may be glucose and the carbon source of the feed may be sucrose.

Sterilization of the culture medium may or may not be performed. Sterilization of the culture medium may be done to prevent bacterial contamination (contamination). Sterilization of the culture medium may be paraphrased as sterilization or sanitization. Sterilization methods include sterilization under high temperature and pressure conditions, sterilization by UV irradiation, and sterilization using filters or membranes. Sterilization of the culture medium may be done batchwise or continuously. For example, autoclave sterilization or batch sterilization in a culture tank are examples of methods in which sterilization under high temperature and high pressure conditions is performed batchwise. For example, the method in which sterilization under high temperature and high pressure conditions is performed continuously includes continuous sterilization equipped with a plate heat exchanger. Sterilization of sugar may be performed simultaneously with other culture medium components or separately from other components. Preferably, sugar and other components may be sterilized separately.

The concentration of the carbon source in the medium is not limited as long as the bacterium can grow and produce L-glutamic acid. The concentration of the carbon source in the medium may, for example, be as high as possible to the extent that the production of L-glutamic acid is not inhibited. The concentration of the carbon source in the medium may be, for example, 1 to 50 w/v%, preferably 1 to 30 w/v%, more preferably 3 to 10 w/v%, as a starting concentration (concentration in the initial medium). Additional carbon sources may be added to the medium as appropriate. For example, an additional carbon source may be added to the medium as the carbon source is consumed as the fermentation progresses. In fed-batch or continuous culture, the amount of carbon source supplied may be the amount that results in a sufficiency condition (a condition in which an excess amount of carbon source is supplied over the amount (capacity) of the bacterium of the invention) or a limitation condition (a condition in which an insufficient amount of carbon source is supplied as compared to the amount (capacity) of carbon utilization of the bacterium of the invention).

. Cultures may be conducted under aerobic or microaerophilic conditions using, for example, liquid medium. Aerobic conditions" means that the concentration of dissolved oxygen in the liquid medium is 0.33 ppm or more which is the detection limit of an oxygen membrane electrode, preferably 1.5 ppm or more. The oxygen concentration under aerobic conditions may be controlled to, for example, 5-50% of the saturated oxygen concentration, preferably 10%. The term "microaerophilic conditions" may mean conditions in which the concentration of dissolved oxygen in the medium is less than 0.33 ppm. For example, the concentration of dissolved oxygen in the medium under microaerophilic conditions may be less than 0.30 ppm, 0.25 ppm, 0.20 ppm, 0.15 ppm, 0.10 ppm, or 0.05 ppm. The oxygen concentration under microaerophilic conditions may be controlled, for example, to less than 5%, 3.75%, 3.125%, 2.5%, 1.875%, 1.25%, or 0.8125% of saturated oxygen concentration. Cultures may be aerated, shaken, agitated, or a combination thereof. The pH of the culture medium may be, for example, pH 3 to 10, preferably pH 4.0 to 9.5. During culture, the pH of the medium can be adjusted as needed. The pH of the medium can be adjusted using various alkaline or acidic substances such as ammonia gas, ammonia water, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 40°C, preferably 25 to 37°C. In main culture, the culture temperature may be varied in two or more steps. For example, as disclosed in Journal of Industrial Microbiology & Biotechnology (2002) 28, 333-337, the culture temperature may be shifted to a higher temperature from 33°C to 37-40°C. The culture period may be, for example, 10 to 120 hours. The culture may continue, for example, until the carbon source in the medium is consumed or until the activity of the bacterium of the invention ceases. Culturing the bacterium of the present invention under these conditions results in the accumulation of L-glutamic acid in the medium and/or in the cells of bacterium.

. The culture can also be performed while precipitating L-glutamic acid in the medium using a liquid medium adjusted to the conditions under which L-glutamic acid precipitates. Such conditions under which L-glutamic acid precipitates include conditions of pH5.0 to 4.0, preferably pH 4.5 to 4.0, more preferably pH 4.3 to 4.0, and especially preferably pH 4.0 (EP1078989A). When a liquid medium adjusted to the conditions under which L-glutamic acid precipitates is used, pantothenic acid can be added to the medium for more efficient crystallization (WO2004/111258). When a liquid medium adjusted to the conditions for precipitation of L-glutamic acid is used, crystals of L-glutamic acid can be added to the medium as seed crystals for more efficient crystallization (EP1233069A). When a liquid medium adjusted to the conditions for precipitation of L-glutamic acid is used, crystals of L-glutamic acid and L-lysine can be added to the medium as seed crystals for more efficient crystallization (EP 1624069A).

The fermentation liquid can be processed, for example, in a liquid cyclone. The liquid cyclone can be, for example, made of ceramic, stainless steel, or resin, with a general shape and a cylindrical diameter of 10 to 110 mm. The feed volume of the fermentation liquid into the liquid cyclone can be set, for example, according to the concentration of bacterium and L-glutamic acid in the fermentation liquid. The feed rate of the fermentation liquid to the liquid cyclone may be, for example, 2 to 1200 L/min.

. The formation of L-glutamic acid can be confirmed by known methods used to detect or identify the compound. Such methods include, for example, HPLC, LC/MS, GC/MS, and NMR. These methods can be used alone or in combination as appropriate.

. The collection of L-glutamic acid from the fermentation liquid can be performed by known methods used for separation and purification of compounds. Such methods include, for example, the ion exchange resin method (Nagai, H. et al., Separation Science and Technology, 39(16), 3691-3710), precipitation method, membrane separation method (JP H9-164323 A, JP H9-173792 A), crystallization precipitation method (WO2008/078448, WO2008/078646), and crystallization method (WO2008/078448, WO2008/078646). These methods can be used alone or in combination as appropriate. If L-glutamic acid accumulates in the cells of bacterium, L-glutamic acid can be recovered from the supernatant obtained by crushing the bacterial cells by ultrasonic waves and removing the bacterial cells by centrifugation, for example, using an ion exchange resin method. The recovered L-glutamic acid may be the free form, a salt thereof, or a mixture thereof. The salts include, for example, sulfate, hydrochloride, carbonate, ammonium, sodium, and potassium salts. Specifically, for example, free L-glutamic acid, sodium L-glutamate (e.g. monosodium L-glutamate; MSG), ammonium L-glutamate (e.g. monoammonium L-glutamate) or mixtures thereof. For example, monosodium L-glutamate (MSG) is obtained by crystallizing ammonium L-glutamate in the fermentation liquid with acid and adding an equimolar amount of sodium hydroxide to the crystals. Monosodium L-glutamate crystals can be used, for example, as an umami seasoning. Monosodium glutamate crystals may be used as a seasoning by mixing with nucleic acids such as sodium guanylate and sodium inosinate, which also have umami taste.

If L-glutamic acid precipitates in the medium, it can be recovered by centrifugation or filtration. L-glutamic acid precipitated in the medium may also be isolated after crystallization of L-glutamic acid dissolved in the medium.

The recovered L-glutamic acid may contain components other than L-glutamic acid, such as bacterial cells, culture medium components, water and bacterial metabolic byproducts, etc. The L-glutamic acid may be purified to the desired degree. The purity of the recovered L-glutamic acid may be, for example, greater than 50% (w/w), preferably greater than 85% (w/w), and especially preferably greater than 95% (w/w) (JP1214636B, U.S. Patent No. 5431933, U.S. Patent No. 4956471, US Patent No. 4777051, US Patent No. 4946654, US Patent No. 5840358, US Patent No. 6238714, US Patent Application Publication No. 2005/0025878 ).

### Examples

The invention is described more specifically below with reference to the non-limiting examples.

### <1> Construction of a modified strain of Corynebacterium glutamicum

### <1-1> Construction of a vector for introducing wild-type acetyl CoA hydrolase gene

Using a chromosomal DNA of C. *glutamicum* ATCC 13869 (strain 2256) carrying the wild-type acetyl CoA hydrolase gene as a template, a DNA fragment containing the wild-type acetyl CoA hydrolase gene was amplified by PCR using primers of SEQ ID NOs: 5 and 6. By linking the amplified DNA fragments with BamHI and PstI-digested pVK9 (U.S. Patent Application Publication No. 2006/0141588) by an infusion reaction, a vector for introducing the wild-type acetyl-CoA hydrolase gene (pVK9-Acetyl-CoA hydrolase (WT)) was obtained.

The nucleotide sequence of the wild-type acetyl CoA hydrolase gene and the amino acid sequence of the wild-type acetyl CoA hydrolase encoded by the gene are shown in SEQ ID NO: 1 and SEQ ID NO: 2, respectively.

### <1-2> Construction of a vector for introducing a mutant acetyl CoA hydrolase gene

A chromosomal DNA of a derivative strain of *C*. *glutamicum* ATCC 13869 (strain 2256) containing a mutant acetyl CoA hydrolase gene having a mutation (S383C) that replaces a serine residue at position 383 of wild-type acetyl CoA hydrolase with a cysteine residue was used as the template. A DNA fragment containing the mutant acetyl CoA hydrolase gene was amplified by PCR using primers of SEQ ID NO: 5 and SEQ ID NO: 6. A vector for introducing the mutant acetyl-CoA hydrolase gene (pVK9-Acetyl-CoA hydrolase (S383C)) was obtained by linking the amplified DNA fragment with BamHI and PstI-digested pVK9 by an infusion reaction.

The nucleotide sequence of the mutant acetyl CoA hydrolase gene and the amino acid sequence of the mutant acetyl CoA hydrolase encoded by the gene are shown in SEQ ID NOs: 3 and 4, respectively.

### <1-3> Construction of a modified strain of Corynebacterium glutamicum

*C. glutamicum* 2256ΔsucAΔldhA yggB* strain (WO2014/185430) was transformed with the constructed mutant transgene vector. From the transformants obtained, according to the method described in WO2006/057450, the wild-type acetyl CoA hydrolase gene-introduced strain and the mutant acetyl CoA hydrolase gene-introduced strain were selected.

Each of the constructed wild-type acetyl-CoA hydrolase gene transfer vector (pVK9-Acetyl-CoA hydrolase (WT)) and the mutant acetyl-CoA hydrolase gene transfer vector (pVK9-Acetyl-CoA hydrolase (S383C)) was singly introduced into *C. glutamicum* 2256ΔsucAΔldhA yggB* strain to obtain the wild-type acetyl CoA hydrolase gene-introduced strain and the mutant acetyl CoA hydrolase gene-introduced strain. A control strain was also obtained by introducing pVK9 alone into the *C*. *glutamicum* 2256ΔsucAΔldhA yggB* strain.

The *C*. *glutamicum* 2256ΔsucAΔldhA yggB* strain is an L-glutamic acid producing strain derived from *C. glutamicum* 2256 (ATCC 13869), lacking the ldhA and sucA genes and having an IS mutation (V419::IS).

### <2>L-glutamic acid production culture

L-glutamic acid production culture was conducted using each of the constructed strains (i.e., the wild-type acetyl CoA hydrolase gene-introduced strain, the mutant acetyl CoA hydrolase gene-introduced strain and control strain). The composition of the culture medium used is shown in Table 1.

**[Table 1]**

| Table 1 Meium composition | |
|---|---|
| Glucose | 80 g/L |
| (NH₄)₂SO₄ | 30 g/L |
| KH₂PO₄ | 1 g/L |
| MgSO₄ · 7H₂O | 0.4 g/L |
| FeSO₄ · 7H₂O | 0.01 g/L |
| MnSO₄ · 5H₂O | 0.01 g/L |
| VB₁ | 200 µg/L |
| Biotin | 60 µg/L |
| Mameno | 0.48 g/L |
| CaCO₃ | 50 g/L |

| | |
|---|---|
| The medium having the above composition was prepared and adjusted to pH8.0 with KOH, followed by sterilized by autoclave (121°C, 20min). To the sterilized medium, calcium carbonate dry-heat sterilized (180°C, 6 hours) was added at the final concentration of 50g/L, and used for the culture. | |

Each strain was inoculated into 20 mL of the above medium (containing 50 g/L calcium carbonate) contained in a 500 mL Sakaguchi flask and incubated at 31.5°C in a box shaker (ABLE ML-190) with shaking at 120 rpm. After 25 hours of culture, the culture medium was sampled. 0.05 ml of the sampled culture medium was diluted 100-fold by adding it to 4.95 ml of 0.1N HCl solution and the absorbance (OD) at 620 nm was measured. The sampled culture medium was centrifuged at 15,000 rpm for 1 min and the concentration of L-glutamic acid in the supernatant was determined using a Biotech Analyzer BF7 (Oji Instrument Co., Ltd.) to calculate the yield of L-glutamic acid per sugar.

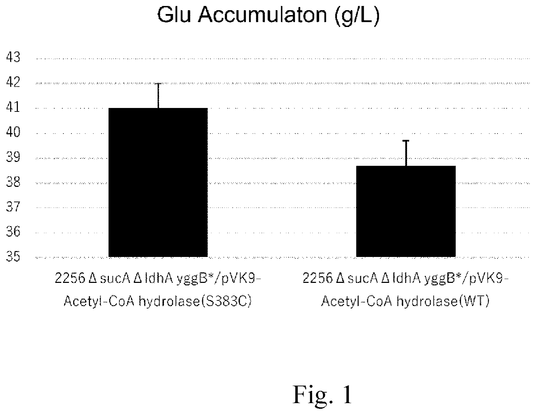

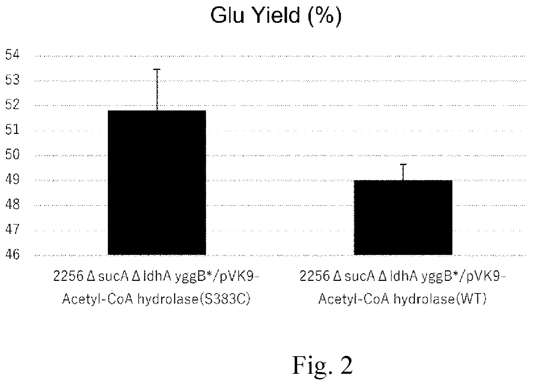

Results are shown in Figures 1 and 2. The mutant acetyl CoA hydrolase gene-introduced strain showed higher L-glutamate accumulation and L-glutamate yield per sugar than the wild-type acetyl CoA hydrolase transgenic strain (Figures 1 and 2).

### Industrial applicability

According to the present invention, the L-amino acid production ability of the coryneform bacterium can be improved and L-amino acids can be produced efficiently.

### Sequence Table Description

SEQ ID NO: 1: wild-type acetyl CoA hydrolase gene sequence of *Corynebacterium glutamicum* 2256 (ATCC 13869)
SEQ ID NO: 2: amino acid sequence of wild-type acetyl CoA hydrolase from *Corynebacterium glutamicum* 2256 (ATCC 13869)
SEQ ID NO: 3: Nucleotide sequence of the mutant acetyl CoA hydrolase gene of *Corynebacterium glutamicum* 2256 (ATCC 13869)
SEQ ID NO: 4: amino acid sequence of mutant acetyl CoA hydrolase from *Corynebacterium glutamicum* 2256 (ATCC 13869)
SEQ ID NO: 5 and Sequence No. 6: Primers
SEQ ID NO: 7: sequence of the wild-type yggB gene of *Corynebacterium glutamicum* 2256 (ATCC 13869)
SEQ ID NO: 8: amino acid sequence of the wild-type YggB protein of *Corynebacterium glutamicum* 2256 (ATCC 13869)
SEQ ID NO: 9: sequence of mutant yggB gene (V419::IS)
SEQ ID NO: 10: amino acid sequence of mutant YggB protein (V419::IS)

## Claims

1. A coryneform bacterium having L-glutamic acid producing ability, wherein said coryneform bacterium has been modified to harbor a mutant acetyl CoA hydrolase gene encoding a mutant acetyl CoA hydrolase having the substitution of a serine residue at position 383 in the amino acid sequence of the wild-type acetyl CoA hydrolase with another amino acid residue.

2. The coryneform bacterium according to claim 1, wherein the other amino acid is lysine, glutamic acid, threonine, aspartic acid, asparagine, glutamine, arginine, cysteine, histidine or methionine.

3. The coryneform bacterium according to claim 1, wherein the other amino acid is cysteine.

4. The coryneform bacterium according to any one of claims 1 to 3, wherein the wild-type acetyl CoA hydrolase is a protein as described in (a), (b) or (c) below.
(a) a protein comprising the amino acid sequence shown in SEQ ID NO: 2;
(b) a protein comprising an amino acid sequence including a substitution(s), a deletion(s), an insertion(s) and/or an addition(s) of 1 to 10 amino acid residues in the amino acid sequence shown in SEQ ID NO: 2, and having acetyl CoA hydrolase activity;
(c) a protein comprising an amino acid sequence having 90% or more identity to the amino acid sequence shown in SEQ ID NO: 2 and having acetyl CoA hydrolase activity.

5. The coryneform bacterium of any one of claims 1 to 4, wherein the coryneform bacterium is a bacterium belonging to the genus *Corynebacterium.*

6. The coryneform bacterium of any one of claims 1 to 4, wherein said coryneform bacterium is *Corynebacterium glutamicum.*

7. The coryneform bacterium of any one of claims 1-6, wherein said coryneform bacterium has been further modified to harbor a mutant yggB gene, wherein the mutant yggB gene is a gene encoding a protein comprising an amino acid sequence including a substitution(s), a deletion(s), an insertion(s) and/or an addition(s) of one or several amino acids in the amino acid sequence of SEQ ID NO: 8.

8. The coryneform bacterium according to claim 7, wherein the mutant yggB gene is a gene encoding a protein comprising the amino acid sequence of SEQ ID NO: 10.

9. A method for the production of L-glutamic acid, comprising
culturing the coryneform bacteria of any one of claims 1 to 8 in a culture medium to accumulate L-glutamic acid in said medium and/or cells of the bacterium, and
collecting L-glutamic acid from the culture medium and/or the cells of the bacterium.

10. A mutant acetyl CoA hydrolase having the substitution of a serine residue at position 383 in the amino acid sequence of wild-type acetyl CoA hydrolase with another amino acid residue.

11. The mutant acetyl CoA hydrolase according to claim 10, wherein the other amino acid is lysine, glutamic acid, threonine, aspartic acid, asparagine, glutamine, arginine, cysteine, histidine or methionine.

12. The mutant acetyl CoA hydrolase according to claim 10, wherein the other amino acid is cysteine.

13. The mutant acetyl CoA hydrolase according to any one of the claims 10 to 12, wherein the protein encoded by the acetyl CoA hydrolase gene is a protein as described in (a), (b) or (c) below.
(a) a protein comprising the amino acid sequence shown in SEQ ID NO: 2;
(b) a protein comprising an amino acid sequence including a substitution(s), a deletion(s), an insertion(s) and/or an addition(s) of 1 to 10 amino acid residues in the amino acid sequence shown in SEQ ID NO: 2 and having acetyl CoA hydrolase activity;
(c) a protein comprising an amino acid sequence having 90% or more identity to the amino acid sequence shown in SEQ ID NO: 2 and having acetyl CoA hydrolase activity.

14. A mutant acetyl CoA hydrolase gene encoding the mutant acetyl CoA hydrolase according to any one of claims 10 to 13.
